# EUROPEAN PATENT APPLICATION

(11) **EP 0 919 184 A1**
(43) Date of publication of application: **02.06.1999**
(21) Application number: 98309569.6
(22) Date of filing: 23.11.1998
(51) Int. Cl.: A61B 5/08, A61B 5/113

(54) **Region-of-interest setting apparatus for respiration monitoring and a respiration monotoring system**

(30) Priority: 21.11.1997 JP 321324/97; 13.11.1998 JP 324129/98
(71) Applicant: TOSHIBA ENGINEERING CORPORATION, Kawasaki-shi, Kanagawa-ken 210 (JP)
(72) Inventor: Ishihara, Ken, Takarazuka-shi, Hyogo-ken (JP); Miyake, Yoshio c/o Toshiba Engineering Corp., Kanagawa-ken (JP)
(74) Representative: Blatchford, William Michael

(57) **Abstract**

A region-of-interest (ROI) setting apparatus for respiration monitoring and a respiration monitoring system are capable of setting an ROI in an automatic manner. Absolute values of subtractions between every successive pairs of frame images produced by a CCD camera 2 for a period of time equal to half a respiration period are calculated, then summed and stored. Based on the change information thus calculated and stored, the position and size of each changed region is calculated. Provisional regions are set from the changed regions successively from the greatest to the smallest one thereof. In each of these provisional regions, processing is carried out to determine whether a grey level histogram indicative of distribution of the number of pixels for respective grey level values includes a twin peak characteristic having twin peaks each equal to or higher than a predetermined value, and whether the area of the corresponding changed region is equal to or greater than a predetermined value. One of the provisional regions that satisfies these conditions is set as an ROI.

## Description

The present invention relates to a respiration monitoring system which is capable of continuously monitoring the respiratory state of a person, involving, for instance, visual sensing of a patient in a non-restrictive manner. The invention also relates to apparatus for setting a region of interest for respiration monitoring.

In recent years, the number of persons of advanced age has increased rapidly and it is desired to increase the number of staff who take care of bedridden elderly persons with attendant increasing personnel costs and medical care expenditure. This is an object of great public concern.

Respiration monitoring systems have been developed which are capable of continuously monitoring the respiratory state of a bedridden person through visual sensing in a non-restrictive manner.

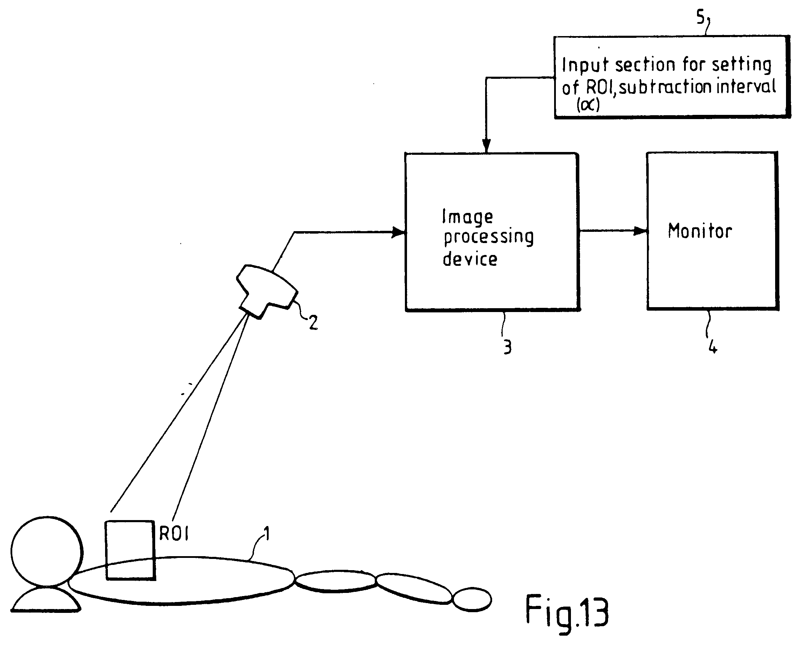

Fig. 13 shows a typical conventional respiration monitoring system as disclosed in "Automatic Measurements of the Number of Respirations under Complete Non-restrictions by a Visual Sensing System" reported in the Proceedings of the 16th Bio-Mechanism Society Conference held on November 25, 1995 by the Bio-Mechanism Society (pages 279 to 282). This respiration monitoring system includes a CCD camera 2 for imaging or photographing the chest of a patient 1 as an object to be imaged who is lying on a bed, and an image processor 3 connected to an output of the CCD camera 2 for successively extracting an inter-image difference or subtraction (i.e., subtraction between every two successive images) of continuous motion pictures at a constant rate. Predetermined image processing steps are performed so as to obtain or calculate a difference component thereby to measure the number of respirations per unit time. The system also includes a monitor 4 connected to an output of the image processor 3 for displaying a visual indication of the detected data including the measured number of respirations per unit time, the state of respiration and/or for displaying an abnormal state in the case of an abnormality taking place. A setting input stage 5 allows an operator to input various settings such as a processing region in the form of a region of interest (hereinafter simply referred to as "ROI") of the image processor 3, a subtraction interval (α) in the image processing, etc.

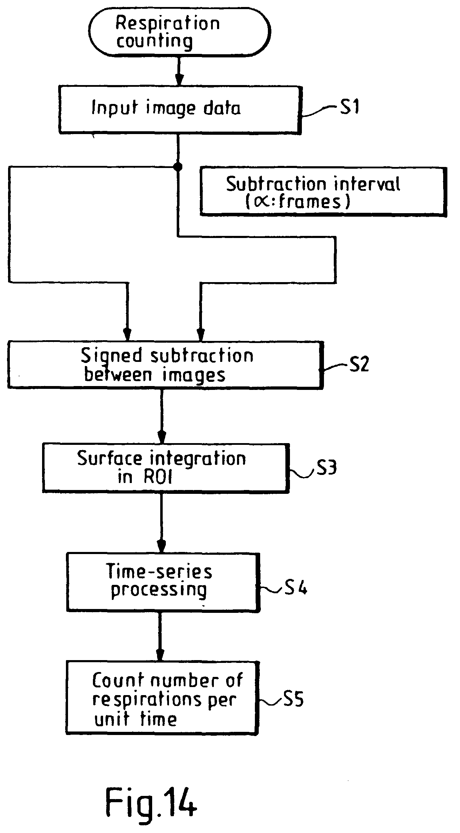

Fig. 14 is a flow chart showing operation (i.e., respiration counting operation) of the above-mentioned conventional respiration monitoring system. Firstly in step S1, the image processor 3 successively receives image data from the CCD camera 2, and in step S2, it performs signed (+, -) subtraction of pixels between every adjacent two images that are spaced from each other by a predetermined frame interval (α). Subsequently, in step S3, the image processor 3 performs surface integration within a ROI as follows. The extracted image data are subjected to signed subtraction (i.e., subtraction with a plus or minus sign) to provide binarized images, from which an area of variations for each image is calculated. Each area thus obtained is then surface integrated in terms of brightness to provide a signed rate of change for each changed portion. In step S4, the signed rate of change is processed in a serial manner each time the CCD camera 2 takes one frame to produce a result on which respiration counting is carried out in step S5.

In this manner, the conventional respiration monitoring system can monitor the state of respiration through visual sensing in a non-restrictive and continuous manner.

With this prior system, however, a supervisor who also acts as an operator has to input manually a specific region of interest (ROI) setting among the pictures or images photographed by the CCD camera 2, for which region image processing is to be performed for measuring or counting the number of respirations per unit time, while watching the monitor 4, referred to above.

Thus, with the conventional system in which the ROI setting is input manually, if, for instance, a patient to be monitored or photographed has moved to displace the ROI, it is difficult to measure the number of respirations per unit time in an accurate manner. In practice respiration measurement becomes impossible when such a situation is left unattended. As a result, it is impossible to measure automatically the respiration rate for a long period of time. On the other hand, if the deviation or displacement of the ROI (i.e., movement of the person) is monitored by a supervisor to allow long-term accurate respiration counting, personnel expense and the amount of labour cannot be reduced.

Moreover, in this case, a change in the value of surface integration becomes small depending upon the ROI setting, so the accuracy of respiration counting can be reduced due to noise or other interference, or becomes difficult to recognise inhalation and exhalation of the person on a monitor.

Furthermore, with the conventional respiration monitoring system, the frame interval α for subtraction is also set and input by the operator, so similarly to the setting of the ROI, this gives rise to difficulty in long-term respiration counting. The frame interval α is input by the operator based on his or her experience, so the accuracy of respiration counting depends on the setting of α and sometimes decreases due to an inappropriate setting, with the result that recognition of inhalation and exhalation on the monitor becomes difficult.

Further, the conventional respiration monitoring system calculates the rate of change in the darkness or grey level within the ROI using an inter-image subtraction with a plus or minus sign, and hence, if an increase and a decrease of brightness within the ROI are equal to each other in their areas, it is determined that there is no change in the rate.

According to a first aspect of the present invention, there is provided region-of-interest setting apparatus for respiration monitoring comprising:
imaging means (such as a camera) for imaging an object to be monitored;
provisional-region setting means for extracting changed portions between successive images taken by the imaging means and setting an optimal one of the thus extracted changed portions as a provisional region;
determining means (e.g. an optimal-ROI determining stage) for determining whether the provisional region set by the provisional-region setting means can be adopted as a changed region for respiration counting; and
region-of-interest setting means (e.g. an ROI setting and storing stage) for setting the provisional region as a region of interest for respiration counting when the determining means determines that the provisional region can be adopted as a changed region for respiration counting.
With the above arrangement, it is possible to set the region of interest in an automatic manner.

In a preferred embodiment, the provisional-region setting means comprises:
subtracted-image storing means (e.g., an original image memory and a subtraction and summation circuit) for calculating absolute values of subtractions between every successive two of a plurality of frame images taken during at least half a respiration period, and for summing and storing the absolute values of subtractions of the images thus obtained for predetermined frames; and
region-of-interest candidate setting means for calculating the position and size of each of the changed regions based on brightness information of each subtracted image stored in the subtracted-image storing means and setting at least the greatest among the changed regions as a provisional region.

With the above arrangement, for a candidate of the region of interest, a single region can be selected in which there can be seen changes over the largest area, and hence it is possible to select as an ROI candidate a region for which a sufficient respiration measuring or counting accuracy can be ensured in the presence of noise.

In another preferred embodiment, the region-of-interest candidate setting means (e.g., a candidate-region extracting stage) is arranged to calculate the position and size of each of the changed regions based on brightness (change) information of each subtracted image stored in the subtracted-image storing means and to select an optimal one of the changed regions successively from the greatest to the smallest thereof as a provisional region.

With the above arrangement, in cases where a large region is not used for setting a region of interest, the next candidate can be retrieved, thus obviating the necessity of again performing a provisional region setting operation. This leads to an enhancement in processing.

In a further preferred embodiment, the determining means determines whether in the provisional region set by the provisional-region setting means, a grey level histogram indicative of distribution of the number of pixels for respective grey level values includes a twin peak characteristic having twin peaks each equal to or higher than a predetermined value. In one embodiment, the determining means comprises an optimal-ROI determining stage, and the predetermined value is set in such a manner that peaks of the twin-peak characteristic can be detected in the presence of noise.

In this way, on the basis of the grey level histogram having a vertical difference (i.e., difference between the highest and lowest points) not less than a predetermined value, it is possible to select, among the ROI candidates, an optimal region of interest in which a sufficient difference in contrast can be detected to provide a satisfactory accuracy in optical measurements. In this case, since the twin peak characteristic is a required condition, reliability in maintaining measuring accuracy can be further enhanced.

In a yet further preferred form the determining means (an optimal-ROI determining stage) determines whether the area of the changed region is equal to or greater than a predetermined value. This allows not only selection of an optimal region of interest for which sufficient measuring accuracy can be obtained in the presence of noise, but also easily to recognise variations in exhalation and inhalation of the object such as a patient on a monitor for example.

In a still further preferred form of, when the determining means (an optimal-ROI determining stage) determines that the provisional region set by the provisional region setting means cannot be adopted as a changed region for respiration counting, the provisional region setting means again performs the provisional region setting. Accordingly, the process of setting a region of interest is carried out repeatedly until an optimal region of interest is set, thereby increasing reliability in ROI setting.

In a further preferred form, the apparatus further comprises region-of-interest setting starting-condition determining means (e.g., an object detecting stage) for detecting, as a condition for starting the setting of a region of interest, whether the object to be monitored has come into a field of view of the imaging means.

With this arrangement, even if the patient has moved away from the bed, when the patient returns, a region of interest is automatically set once again, so respiration monitoring can be resumed in an automatic manner.

According to a second aspect of the invention, there is provided a respiration monitoring system comprising:
imaging means for imaging an object to be monitored;
setting means for setting a region of interest for respiration counting in images taken by a imaging means;
change detecting means (e.g., an ROI image extracting stage, a previous-frame memory, a frame-memory selecting stage, an inter-image subtraction stage and a surface integration stage 25) for calculating absolute values of subtractions between successive images produced by the imaging means in the region of interest set by the setting means and for summing up the absolute values of subtractions for every predetermined number of frames;
counting means (e.g., a serial data storing stage and a respiration calculating stage) for counting the number of respirations per unit time based on a trend of time-series changes obtained by the change detecting means.

With the above arrangement, the absolute values of subtractions between successive images are summed for every predetermined number of frames, so that even if the brightness in the region of interest changes with the area of increasing brightness equal to the area of decreasing brightness, such changes can be detected correctly, thus preventing errors in measurements which would otherwise result from failure to detect the changes as in the prior art.

In a preferred form, the system further comprises condition determining means for determining, based on a frequency analysis of a trend of time-series data, whether the state of respiration is normal.

With this arrangement, discrimination can be clearly made as to whether the trend of time-series data is generated based on noise and the like, or on normal respiration.

In another preferred form, the system further comprises existence determining means for determining whether the object to be monitored is outside a predetermined imaging range. In this way it can be determined whether, for example, a patient to be monitored has moved out of a range to be monitored. This may happen when, for example, the patient gets out of bed for lingering or some other reason, or falls down from the bed. This serves to improve reliability in respiration monitoring.

In a further preferred form, the respiration counting means counts the number of respirations per unit time by measuring the number of peaks in a change of a trend in the time series data, and wherein a threshold is used which is variably set for each peak for counting thereof. Accordingly, the number of respirations per unit time can be reliably counted. That is, the magnitude of each peak occurring in the trend of the time-series data generally changes, and in such a case, if the number of peaks is counted with a constant threshold, there will be a problem that peaks smaller than the threshold cannot be counted. To counter this, the threshold is set according to the magnitude of each peak, so that the number of peaks can be counted correctly irrespective of variations in the peak magnitude.

In a still further preferred form, the system further comprises frame-interval setting means (typically a frame-interval setting stage) for setting the predetermined number based on the summed value calculated by the change detecting means or the number of respirations per unit time counted by the counting means in such a manner as to emphasize changes in grey levels. With this arrangement, measuring accuracy can be improved, and observation of changes on a monitor for example can be effected with ease.

In a yet further preferred form, the means for setting a region of interest for respiration counting in images taken by the imaging means comprises the region-of-interest setting means (an ROI setting stage) of the region-of-interest setting apparatus for respiration monitoring as set out above. With the above arrangement, the entire process of respiration monitoring including the setting of a region of interest can be automated so that respiration of the patient can be monitored for an extended period of time without requiring any human monitoring..

In a further preferred form, the provisional region setting means and/or the determining means operate during respiration counting. Therefore, in cases where the ROI needs to be set again during respiration counting, such new setting can be readily made immediately, thus avoiding interruption of respiration monitoring for a long period of time.

The invention will now be described by way of example with reference to the drawings in which:-
Fig. 1 is a block diagram showing the overall arrangement of a first respiration monitoring system in accordance with the invention;
Fig. 2 is a flow chart showing the overall operation of the respiration monitoring system;
Fig. 3 is a block diagram showing details of an ROI setting stage and a respiration measuring stage.
Fig. 4 is a flow chart showing the operation of the ROI setting stage;
Fig. 5 is a representation of image change information obtained by a subtracting and summing circuit of the system;
Fig. 6 is a grey level histogram;
Fig. 7 is a flow chart showing respiration counting;
Fig. 8 is a time chart showing the respiration speed or rate in terms of the absolute value of subtraction;
Fig. 9 is a block diagram showing an optimal-ROI determining stage of a second respiration monitoring system in accordance with the present invention;
Fig. 10 is a block diagram showing a respiration rate calculating stage of a third respiration monitoring system in accordance with of the invention:
Fig. 11 is a block diagram showing an exemplary method for frequency analysing time-series data in a fourth respiration monitoring system in accordance with the invention;

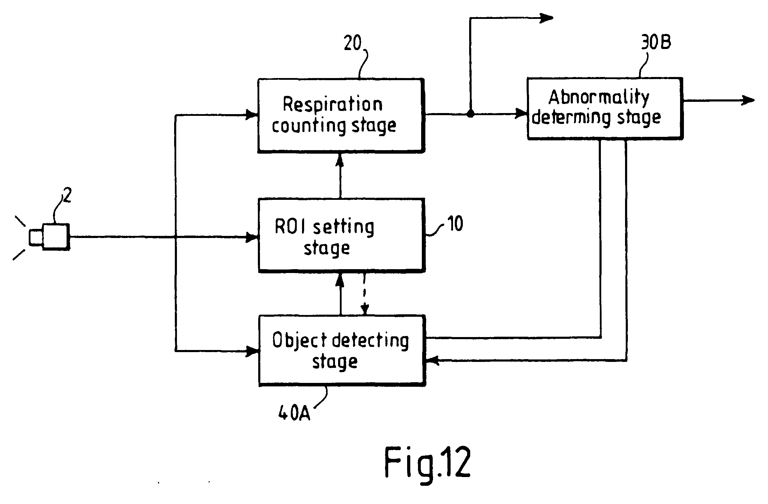

Fig. 12 is a block diagram showing the overall arrangement of a fifth respiration monitoring system in accordance with the invention:
Fig. 13 is a block diagram showing a conventional respiration monitoring system; and
Fig. 14 is a flow chart showing the operation of the conventional respiration monitoring system.

### EMBODIMENT 1

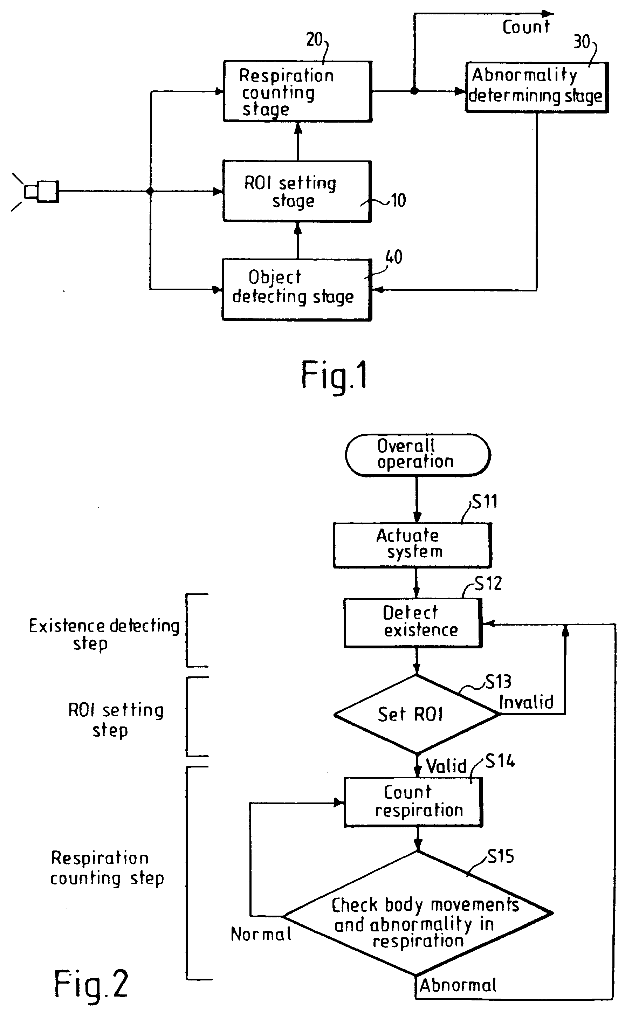

Referring to Fig. 1, a respiration monitoring system comprises a CCD camera 2 for sensing or imaging the chest or thereabouts of a target object such as patient, who is being cared for, a region-of-interest (ROI) setting stage 10 connected to an output of the CCD camera 2 for setting a region of interest, a respiration counting stage 20 connected to an output of the ROI setting stage 10 and the output of the CCD camera 2 for counting the number of respirations per unit time in the region of interest (ROI) which is set by the ROI setting stage 10, an abnormality determining stage 30 connected to an output of the respiration counting section 20 for determining whether the number of respirations per unit time measured is normal or abnormal, and an object detecting stage 40 connected to an output of the abnormality determining stage 30 and the output of the CCD camera 2 for detecting the target object to be monitored.

The object detecting stage 40 detects the target object coming into the view in the CCD camera 2 upon setting of the ROI by continuously detecting a large displacement of the target object on a picture image for a predetermined period of time, and generates a detection or output signal which acts as a trigger to actuate the ROI setting stage 10. Also, when the target object temporarily moves away from a bed or the like, the abnormality determining stage 30 determines an abnormality in the number of respirations per unit time measured, and generates a corresponding output signal, based on which the object detecting stage 40 is started to prepare for watching the bed or thereabouts and again detects the target object when it has returned to the bed, thus reactuating the ROI setting stage 10.

Referring to Fig. 2, in step S11, the system is actuated, and in step S12, the object detecting stage 40 starts to detect the presence and absence of a target object, i.e., whether a target object is on the bed. Upon detection of the target object, the ROI setting stage 10 sets a region of interest (ROI) in step S13. Upon setting of the ROI the ROI setting stage 10 sets a candidate for the ROI and determines the validity or feasibility thereof. If it is determined that the setting of the ROI candidate is invalid, the control process or flow returns to the existence detecting step S12, and the above operations in steps S12 and S13 are repeatedly carried out until a valid ROI candidate is obtained becomes valid.

If the ROI candidate setting is determined to be valid in step S13, the flow then goes to step S14 where respiration counting is carried out. Subsequently, in step S15, if it is determined that the number of respirations per unit time or the movement of the target object (e.g., body movement of a person to be monitored) is abnormal, there may be the possibility that the target object be away from the bed, and thus, the control process returns to the existence detecting step S12, whereas if the determination in step S15 is otherwise (i.e., abnormal), an alarm is generated by the abnormality determining stage 30.

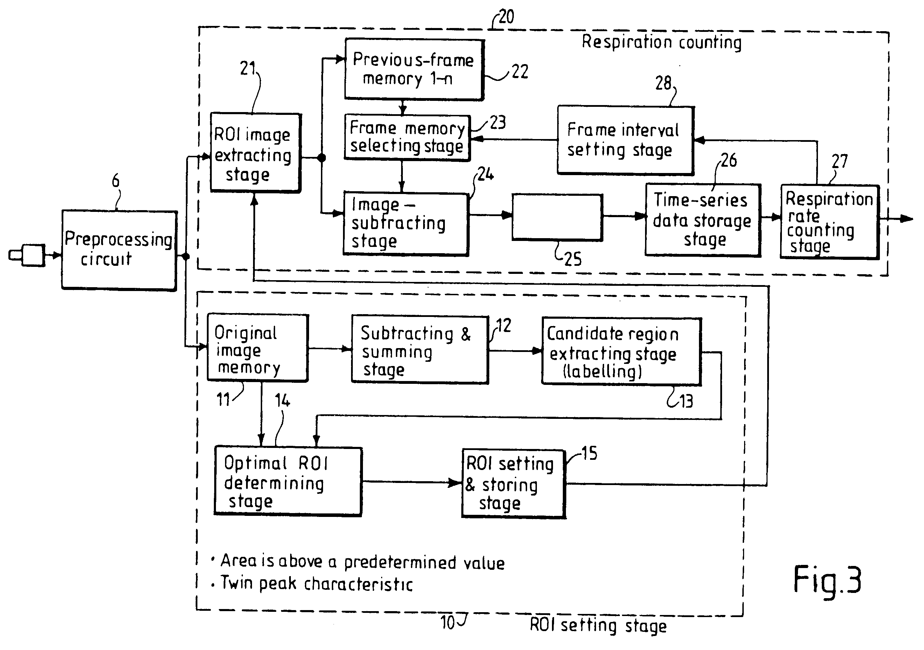

Fig. 3 shows in block form the details of the ROI setting stage 10 and the respiration counting stage 20 of the respiration monitoring system.

The ROI setting stage 10 comprises an original image memory 11 connected to an output of a preprocessing circuit 6 that performs waveform shaping and the like, for continuously taking in the image obtained by the CCD camera 2 for a period of time just half the respiration period. A subtracting and summing circuit 12 is connected to an output of the original image memory 11 for extracting a changed portion of the image stored therein, and a candidate-region extracting stage 13 is connected to an output of the subtracting and summing circuit 12 for labelling the summed image obtained by the subtracting and summing circuit 12 to provide a candidate region for the ROI. An optimal-ROI determining stage 14 is connected to an output of the candidate-region extracting stage 13 and the output of the original image memory 11 for determining whether the region (image region) extracted by the candidate-region extracting stage 13 can be used as the ROI and an ROI setting and storing stage 15 is connected to an output of the optimal-ROI determining stage 14 for setting as the ROI the image region determined to be appropriate or optimal for the ROI by means of the optimal-ROI determining stage 14.

Here, it is to be noted that an operation controller (not shown) is connected to an output of the object detecting stage 40 shown in Fig. 1.

The respiration counting stage 20 comprises an ROI image extracting stage 21 connected to the output of the preprocessing circuit 6 and the output of the ROI setting and storing stage 15 for extracting from the image data obtained by the CCD camera 2 only those image data which lie within the ROI and a previous-frame memory 22 connected to an output of the ROI image extracting stage 21 for storing ROI image data for n frame images. An inter-image subtraction stage 24 is connected to an output of the ROI image extracting stage 21 and an output of a frame memory selecting stage 23 (described later) for performing subtraction between images spaced from each other a distance of α frames. A surface integrates stage 25 connected to an output of the inter-image subtraction stage 24 for surface integrating the values of the inter-image subtractions thereby obtained.

The respiration counting stage 20 comprises a time-series-data storing stage 26 connected to an output of the surface integration stage 25 for storing, as serial data, the values of surface integration obtained by surface integrating the inter-image subtraction in a time serial manner for a predetermined period of time, and a respiration rate calculating stage 27 for calculating the number of respirations per unit time based on the data stored in the time-series-data storing stage 26. A frame interval setting stage 28 is connected to an output of the respiration rate calculating stage 27 for setting a frame interval α with which the inter-image subtraction stage 24 performs inter-image subtraction, based on the result of calculation by means of the respiration rate calculating stage 27, and the frame memory selecting stage 23 is connected to an output of the frame interval setting stage 28 for selecting from the previous-frame memory 22 a previous frame image spaced an interval of a from a current image and outputting it to the inter-image subtraction stage 24.

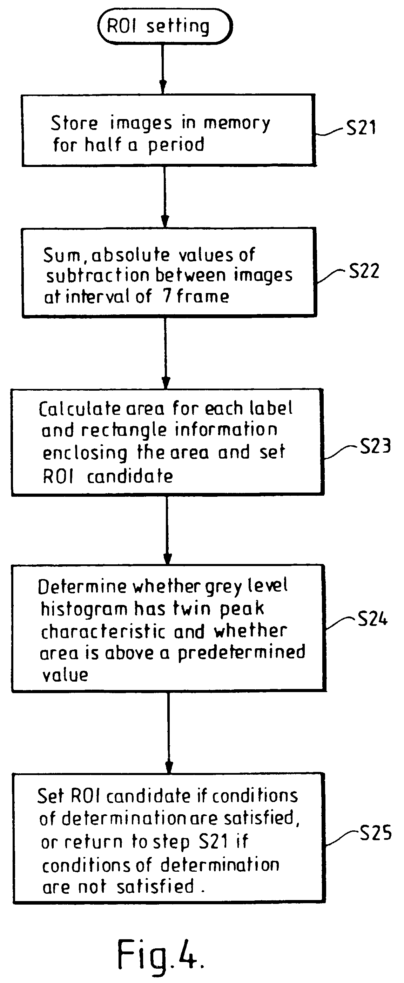

Now, the operation of ROI setting stage 10 will be described with reference to the flow chart of Fig. 4.

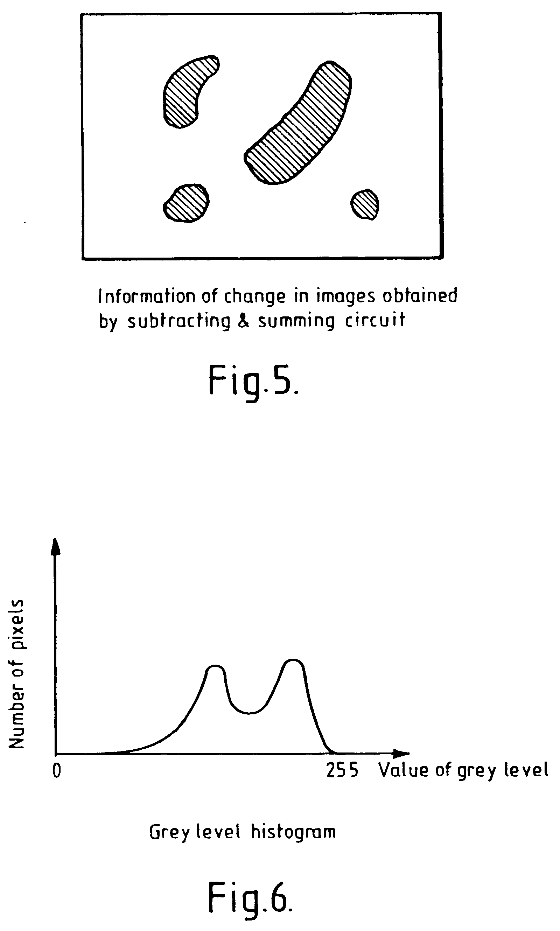

First, in step S21, images from the CCD camera 2 are stored into the original image memory 11 for a period of time equal to half the respiration period. In step S22, the subtracting and summing circuit 12 performs subtraction at a predetermined subtraction interval (e.g., one frame), and sums up information of changes which are given by absolute values of subtractions. An example of such changes of information thus summed is shown in Fig. 5, in which of dark portions indicate image portions for which changes have been recognised during a time equal to half the respiration period. In step S23, the candidate-region extracting stage 13 carries out labelling processing on the information of image changes depicted in Fig. 5, and calculates an area and information of a rectangle enclosing the area for each label, thereby setting an ROI candidate.

Although in this embodiment, a plurality of ROI candidates with labels having gradually decreasing areas from the largest to smallest have been prepared, a single candidate having a label of the largest area can instead be used, and if it is determined according to an optimal-ROI determination to be described later that this candidate is not optimal, the control process returns to step S21 so as to set a new ROI candidate once again.

In step S24, the optimal-ROI determining stage 14 determines an optimal ROI. Specifically, in this optimal ROI determination, an original image date related to the position and size of a rectangular region information set in the first candidate is obtained from the original image memory 11, thus providing a grey level histogram indicative of distribution of the number of pixels at each grey level. The values of grey levels are determined according to 255 steps or degrees of gradation. An example of such a grey level histogram is shown in Fig. 6. In the grey level histogram, it is determined whether a twin peak characteristic including two adjacent peaks at a short interval can be recognised and whether the area of the region (i.e., rectangular region) listed as a candidate is equal to or greater than a predetermined value. If these conditions are not satisfied, the second candidate is taken as the next candidate for its optimality determination according to this embodiment, as referred to above.

Subsequently, if the conditions of determination are satisfied in step S24, the position and size or rectangular area of the optimal ROI are stored in the ROI setting and storing stage 15, and thus the optimal ROI is finally set in step S25. On the other hand, if the conditions of determination are not satisfied, then the control process returns to step S21, thus repeating the optimal ROI setting processing.

After the optimal ROI has been set as described above, the respiration counting stage 20 or counts the number of respirations per unit time using the set ROI.

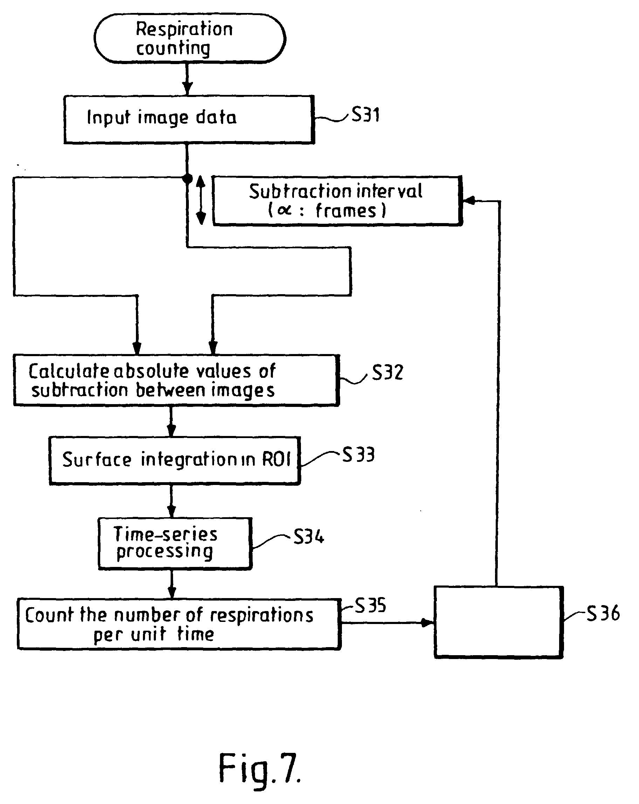

The operation of the respiration counting stage 20 will now be described with reference to Fig. 7.

First, in step S31, image data within the set ROI is fed from the CCD camera 2 to the previous-frame memory 22 and stored therein. Subsequently, in step S32, the inter-image subtraction stage 24 performs inter-image subtraction so as to provide an absolute value of subtraction for each pixel, which is then surface integrated by the surface integration stage 25 in step S33.

The above processes are carried out in a time-series manner, as shown in step S34, and the data obtained during such processings are stored in the time-series-data storing stage 26, based on which data the respiration counting stage 20 counts the number of respirations per unit time in step S35.

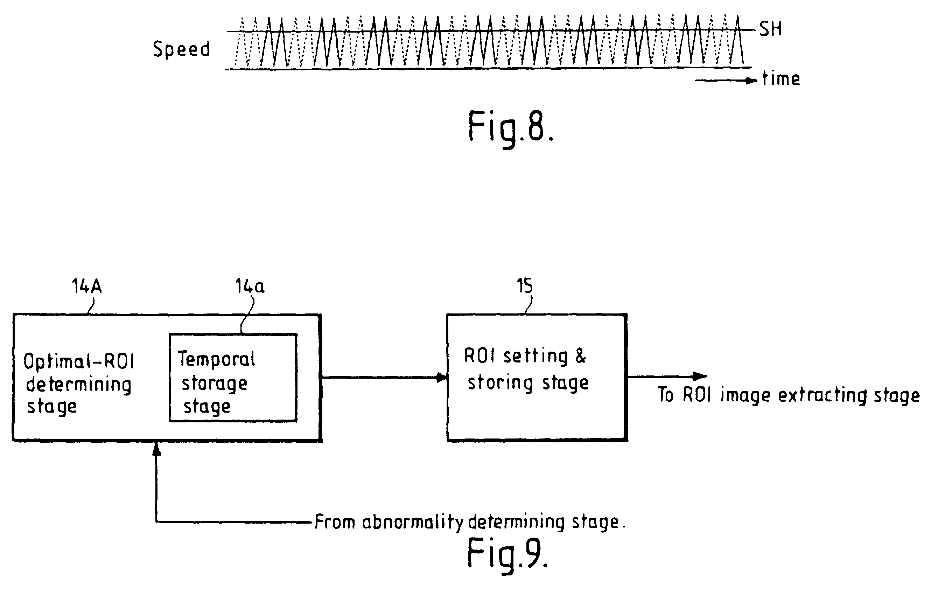

Fig. 8 illustrates in real time the time-series data obtained by the surface integrating stage 25. In this diagram the respiration speed is shown by the absolute value of subtraction, and as will be described, there clearly appear a plurality of twin peaks each having a pair of peaks indicative of exhalation and inhalation, thus emphasising changes or variations despite noise interference..

Here, the number of respirations per unit time is measured by counting the number of peaks exceeding a constant or fixed threshold S_{H}

In step S36, the frame-interval setting stage 28 automatically sets a frame interval in the form of the interval of subtraction α based on the number of respirations per unit time as measured in such a manner as to emphasise changes in the surface integration value (grey level), and outputs it to the frame memory selecting stage 23 which then selects, based on the subtraction interval α, a frame memory having a frame image stored therein previously apart an interval of a frames from the current image, and outputs the data therein to the inter-image subtraction stage 24.

Here, it is to be noted that for the subtraction interval α, a frame interval corresponding for example, to half the respiration period is selected in order to emphasise changes in the grey level or darkness. In view of this, the number of frames stored in the previous-frame memory 22 is selected according to the number of frames per unit time and the respiration period. The previous-frame memory 22, in which the data used for inter-image subtraction was stored, now becomes available for storing the next image data. In cases where the frame interval α is set according to the respiration period (or the number of respirations per unit time), as stated above, a change in the surface integration obtained may be multiplied by 1/α, in order to indicate the surface integration change at the same rate.

### EMBODIMENT 2

In the aforementioned embodiment 1, the respiration counting stage 20 performs respiration counting using the ROI set by the ROI setting stage 10, but in this case, the ROI setting stage 10 may be constructed such that it is always preparing for setting a new ROI during respiration counting by the respiration counting stage 20. In this case, as shown in Fig. 9, an optimal-ROI determining stage 14A is provided with a temporal storage stage 14a for storing an ROI each time the ROI is determined to be optimal by means of the optimal-ROI determining stage 14A. Thus, for example, if the abnormality determining stage 30 determines the presence of abnormality, the ROI setting stage 10 can immediately set again the optimal ROI stored in the temporal storage stage 14a in place of the current ROI set in the ROI setting and storing stage 15. In this manner, it is possible to avoid interrupting respiration counting for the time required to freshly set a new ROI thereby enabling respiration counting to restart immediately. If it is again determined that the number of respirations per unit time freshly counted is abnormal, the abnormal respiration is immediately decided to be not due to inappropriate setting of the ROI thus enabling speedy recovery treatments to be taken.

### EMBODIMENT 3

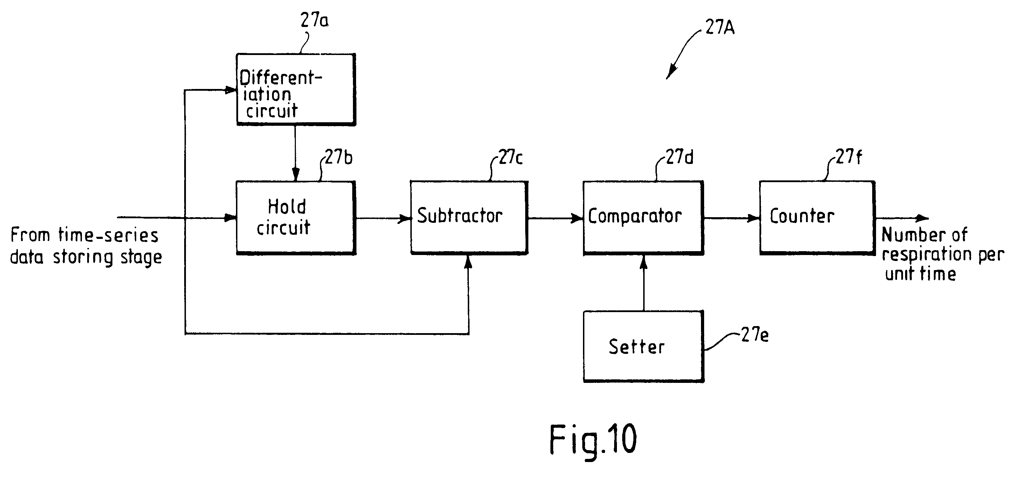

Although in the aforementioned embodiment 1, the respiration rate calculating stage 27 counts the number of peaks using the constant or fixed threshold S_{H}, as shown in Fig. 8, the threshold S_{H} may be varied for each peak. Fig. 10 shows in block form an exemplary arrangement of a respiration rate calculating stage 27A in such a case. The respiration rate calculating stage 27A comprises a differential circuit 27a and a hold circuit 27b both connected to an output of a time-series data storing stage 26 (see Fig. 3), a subtractor 27c connected to outputs of the hold circuit 27b and the time-series data storing stage 26, a setter 27e in which a predetermined value is set, a comparator 27d connected to outputs of the subtractor 27c and the setter 27c, and a counter 27f connected to an output of the comparator 27d.

With the above arrangement, the differential circuit 27a differentiates the time-series data stored in the time-series data storing stage 26 to find its peak, which is then output to the hold circuit 27b and there held therein as a peak value. The subtractor 27c calculates the difference between the peak value and the subsequent time-series data, and outputs the calculated difference value to the comparator 27d in which the difference value is compared with the set value in the setter 27e. The comparator 27d generates an output to the counter 27f when the difference value is greater than the set value. The counter 27f counts the number of outputs from the comparator 27d, thereby providing a count of peaks.

In the respiration rate calculating stage 27A, if a peak value varies, the threshold therein is accordingly changed to detect such a varying peak, so that the number of peaks can be counted accurately irrespective of the magnitude of each peak.

### EMBODIMENT 4

In embodiment 1 described above, the abnormality determining stage 30 is constructed such that it makes an abnormality determination based solely on the number of respirations per unit time as counted by the respiration rate calculating stage 27. However, there may sometimes be a case where the abnormality determining stage 30 cannot measure the true respiration (i.e. respiratory movements) correctly, since the ROI once properly set departs from its original or intended position due to movements of the object to be monitored, or the influence of noise affecting respiration counting. In view of this, to determine whether respiration is counted correctly, a frequency spectrum of a time-series data can be obtained for comparison with a frequency spectrum of a normally or correctly measured respiration. Such a comparison can be made using a statistical test of hypothesis.

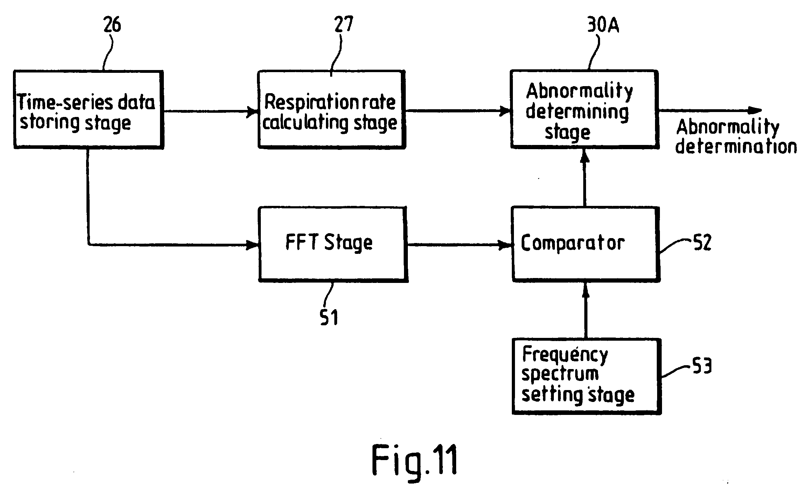

Fig. 11 shows in block form an example of such an arrangement. In this figure, a fast Fourier transform (FFT) stage 51 is provided at an output side of the time-series data storing stage 26 for generating a frequency spectrum of the time-series data output. A comparator 52 compares the frequency spectrum of the time-series data output from the FFT stage 51 with a normal or correct frequency spectrum set in a frequency spectrum setting stage 53. This comparison is made, for example, in terms of the magnitude or strength of a predetermined frequency component. An abnormality determining stage 30A determines, whether the respiration counting is normal based on the comparison of comparator 52.

### EMBODIMENT 5

Although in the above-described embodiment 1, the object detecting stage 40 detects an object coming into or the field of view of the CCD camera 2 upon setting an ROI by detecting a large change in time of the images produced by the camera 2, it may also detect the object going out of the field of view, again by detecting a large change in time of the images. A large change in time of the images can be detected, for example, by detecting a change in contrast or brightness of the images at predetermined time intervals by way of sensing correlation between the images or a change in the grey level histogram thereof. In this embodiment, then, an object detecting stage 40A may be provided with an image memory for the above purpose, but instead the original image memory 11 of the ROI setting stage 10 can be used for investigating correlation between the images, or grey level histograms can be compared with each other at a predetermined time interval using a statistical test of hypothesis so as to perform the above detection. Detection of the object to be monitored having gone out of the field of view is signalled to an abnormality determining stage 30B, so that it is possible to deal with the abnormal condition while discriminating respiratory abnormality from such an abnormal situation in which normal respiration counting can not be effected due to the absence of an object to be watched in the field of view of the camera 2.

As described above, automatic setting of the ROI can provide the advantage of automatic accurate respiration counting for an extended period of time without requiring operator monitoring. In addition, exhalation and inhalation can quite easily be recognised on a monitor.

## Claims

1. A region-of-interest setting apparatus for respiration monitoring comprising:
imaging means for imaging an object to be monitored;
provisional-region setting means for extracting changed portions between successive images produced by the imaging means and selecting one of thus extracted changed portions as a provisional region;
determining means for determining whether the provisional region set by the provisional-region setting means can be adopted as a changed region for respiration counting; and
region-of-interest setting means for setting the provisional region as a region of interest for respiration counting when the determining means determines that the provisional region can be adopted as a changed region for respiration counting.

2. Apparatus according to claim 1, wherein the provisional-region setting means comprises:
subtracted-image storing means for calculating absolute values of subtractions between every successive two of a plurality of frame images taken during at least half a respiration period, and for summing and storing the absolute values of subtractions of the images thus obtained for every predetermined frame; and
region-of-interest candidate setting means for calculating the position and size of each of the changed regions based on brightness information of each subtracted image stored in the subtracted-image storing means and setting at least the greatest among the changed regions as a provisional region.

3. Apparatus according to claim 1, wherein the provisional-region setting means comprises:
subtracted-image storing means for calculating absolute values of subtractions between every successive two of a plurality of frame images taken during at least half a respiration period, and for summing and storing the absolute values of subtractions of the images thus obtained for every predetermined frame; and
region-of-interest candidate setting means for calculating the position and size of each of the changed regions based on brightness information of each subtracted image stored in the subtracted-image storing means and setting an optimal one of the changed regions successively from the greatest to the smallest thereof as a provisional region.

4. Apparatus according to any of claims 1 to 3, wherein the determining means is arranged to determine whether, in the provisional region set by the provisional-region setting means, a grey level histogram indicative of distribution of the number of pixels for respective grey level values includes a twin peak characteristic having twin peaks each equal to or higher than a predetermined value.

5. Apparatus according to claim 4, wherein the determining means is arranged to determine whether the area of the changed region is equal to or greater than a predetermined value.

6. Apparatus according to any of claims 1 to 5, wherein, when the determining means is arranged to determine that the provisional region set by the provisional region setting means cannot be adopted as a changed region for respiration counting, the provisional region setting means is arranged to perform again the setting of a provisional region.

7. Apparatus according to any of claims 1 to 6, further comprising region-of-interest setting starting-condition determining means for detecting, as a condition for starting the setting of a region of interest, whether the object to be monitored has come into a field of view of the imaging means.

8. A respiration monitoring system comprising:
imaging means for imaging an object to be monitored;
setting means for setting a region of interest for respiration counting in images produced by the imaging means;
change detecting means for calculating absolute values of subtractions between successive images produced by the imaging means in the region of interest set by the setting means and for summing the absolute values of subtractions for every predetermined number of frames; and
counting means for counting the number of respirations per unit time based on a trend of time-series changes obtained by the change detecting means.

9. A system according to claim 8, further comprising condition determining means for determining, based on a frequency analysis of a trend of time-series data, whether the state of respiration is normal.

10. A system according to claim 8 or claim 9, further comprising existence determining means for determining whether the object to be monitored has moved out of a predetermined imaging range.

11. A system according to any of claims 8 to 10, wherein the respiration counting means is arranged to count the number of respirations per unit time by measuring the number of peaks in a change of a trend in the time series data, and wherein a threshold is used which is set for each peak for counting thereof.

12. A system according to any of claims 8 to 11, further comprising frame-interval setting means for setting the predetermined number based on the summed value calculated by the change detecting means or the number of respirations per unit time counted by the counting means in such a manner as to emphasise changes in grey levels.

13. A system according to any of claims 8 to 12, wherein the means for setting a region of interest for respiration counting in images taken by the imaging means comprises the region-of-interest setting means of the region-of-interest setting apparatus for respiration monitoring as defined in any of claims 1 to 7.

14. A system according to claim 13, wherein the provisional region setting means and/or the determining means operate during respiration counting.
